# EUROPEAN PATENT APPLICATION

(11) **EP 4 534 546 A1**
(43) Date of publication of application: **09.04.2025**
(21) Application number: 23812217.0
(22) Date of filing: 26.05.2023
(51) Int. Cl.: C07K 7/08, A61K 38/00, A61P 25/28

(54) **NOVEL PEPTIDE AND USE THEREOF**

(30) Priority: 27.05.2022 KR 20220065263
(71) Applicant: Zincure Corp., Seoul 05006 (KR)
(72) Inventor: CHUNG, Sang Won, Seoul 06294 (KR); KIM, Yang-Hee, Seoul 06294 (KR); CHOUNG, Wonken, Seoul 06361 (KR); KIM, Ki-Ryeong, Seoul 02026 (KR); OH, Sang-Seok, Seoul 03021 (KR); LEE, Hyun Seung, Suwon-si Gyeonggi-do 16340 (KR)
(74) Representative: Dehns
(86) International application number: PCT/KR2023/007328
(87) International publication number: WO 2023/229443

(57) **Abstract**

The present invention relates to novel peptides and uses thereof, and more particularly to peptides having a length of 12 to 20 a.a. comprising an amino acid sequence designated as SEQ ID NO: 1 or 2, having the ability to inhibit the formation of abnormal protein aggregates, wherein 1 to 4 of the amino acids excluding glycine are L-type amino acids and the other amino acids are D-type amino acids.

## Description

### TECHNICAL FIELD

The present invention relates to novel peptides and uses thereof, and more particularly to novel peptides based on alloferon and uses in the treatment of neurodegenerative diseases.

### BACKGROUND ART

Zinc is a trace element that is essential for cell proliferation and differentiation and is known as a cofactor in the function and structure of proteins such as enzymes and transcription factors. In addition, the homeostasis of zinc in neurons is known to play an important role in the survival of neurons, and deficiency of zinc in neurons is known to induce apoptosis and cause neurodegenerative diseases such as Alzheimer's disease (AD) and Parkinson's disease (Lien, H. et al, BBRC. 268: 148-154, 2000). Conversely, excessive zinc in neurons are also known to induce cellular damage, which can lead to acute brain injury, such as focal ischemia and seizures (Koh et al., Science. 272: 1013-1016, 1996).

Autophagy is an intracellular mechanism that breaks down organelles during starvation to obtain energy or remove damaged organelles and abnormal or pathological protein aggregates. During autophagy, cytoplasmic components are surrounded by a bilayer membrane and isolated from other organelles to form an autophagosome. At this point, the soluble light chain I (LC3I) in the cytoplasm is converted to light chain II (LC3II), which is attached to the autophagosome membrane. The autophagosome then fuses with the lysosome to form an autolysosome, which is degraded and recycled by several hydrolytic enzymes present in the lysosome.

Recently, it has been shown that protein aggregates seen in neurodegenerative diseases, such as α-synuclein, β-amyloid, tau protein, superoxide dismutase-1 (SOD-1), Huntingtin protein, and TAR DNA-binding protein 43 (TDP-43), can be cleared by promoting autophagy. Indeed, there is evidence that defective autolysosomal function impairs the degradation of those protein aggregates, thereby blocking the flux of autophagic actions, and that this lack of autophagy ultimately leads to the accumulation of neurodegenerative disease byproducts (Zhang et al,. ABBS. 41(6): 437-445, 2009; Lee et al,. Cell. 141(7): 1146-1158, 2010).

Previous studies have shown that the function of lysosomes can be enhanced by zinc supply. On the other hand, it has been also reported that when the potent zinc chelator TPEN is applied to inhibit lysosomal membrane permeabilization (LMP) induced by H₂O₂, tamoxifen, and ethanol, and therefore the autophagic actions are reduced, additional zinc supply can promote autophagy (Lee et al, Glia 57: 1351-1361, 2009; Hwang et al., Biometals 23: 997-1013, 2010; Liuzzi et al., Biol. Trace Elem. Res. 156: 350-356, 2013; Kim et al., Front. Cell. Neurosci. 16: 895750, 2022). Hence, zinc is projected to play an important role in autophagy.

However, no drug has yet been developed to treat neurodegenerative diseases by maintaining zinc homeostasis within neurons.

### DETAILED DESCRIPTION OF THE INVENTION

### TECHNICAL PROBLEM

The present invention aims to address the above problems and others by providing novel peptides and their uses in the treatment of degenerative neurological diseases by maintaining zinc homeostasis in neurons, thereby removing abnormal or pathological protein aggregates such as amyloid beta peptides, tau proteins, and superoxide dismutase, which are pathological substances in degenerative neurological diseases, through autophagy or enhanced lysosomal function.

### TECHNICAL SOLUTION

In one aspect of the present invention, there is provided a novel peptide with a length of 12 to 20 a.a. capable of inhibiting the formation of abnormal protein aggregates, comprising an amino acid sequence designated by SEQ ID NO: 1 or 2, wherein 1 to 4 of the amino acids excluding glycine are L-type amino acids and the other amino acids are D-type amino acids.

In another aspect of the present invention, there is provided a pharmaceutical composition for the treatment of neurodegenerative diseases comprising the peptide above as an active ingredient.

In another aspect of the present invention, there is provided a method of treating a subject suffering from a neurodegenerative disease, comprising administering a therapeutically effective amount of the peptide to the subject.

In another aspect of the present invention, there is provided a method of inhibiting the accumulation of pathogenic protein aggregates in the nervous system of a subject suffering from a neurodegenerative disease, comprising the step of administering a therapeutically effective amount of the peptide to the subject.

### EFFECT OF THE INVENTION

The novel peptides of the present invention, prepared as described above, can be utilized in the development of therapeutics to effectively treat neurodegenerative diseases such as Alzheimer's disease, Parkinson's disease, amyotrophic lateral sclerosis and Huntington's disease by preventing neuronal cell death and regulating intracellular zinc homeostasis. However, the scope of the present invention is not limited by these effects.

### BRIEF DESCRIPTION OF THE DRAWINGS

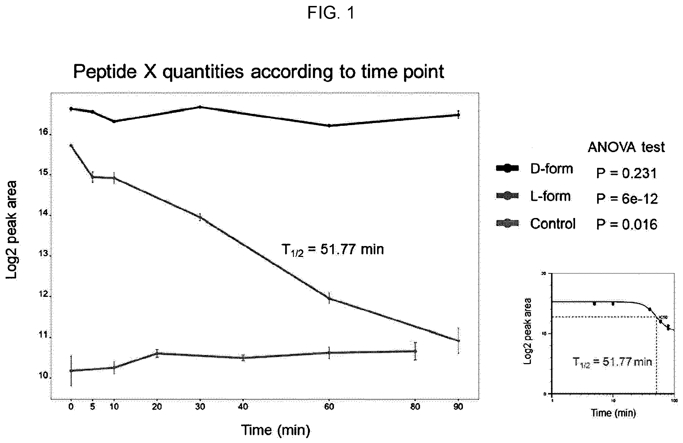

FIG. 1 is a graph showing the results of measuring quantitative changes of alloferon L-type and alloferon D-type peptides by mass spectrometry to investigate their degradation in plasma from mice, where 10 µM of each alloferon peptide was treated in isolated plasma and were incubated at 37°C for 0, 10, 30, 60, and 90 min.

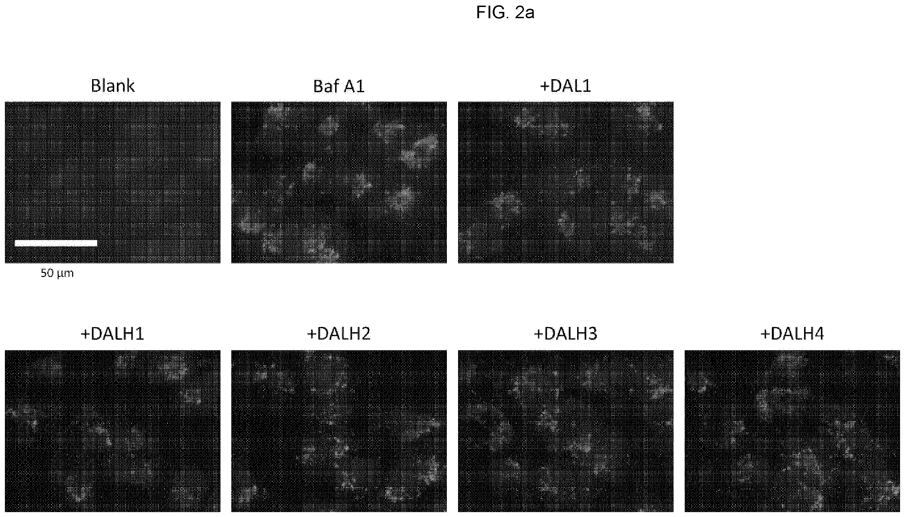

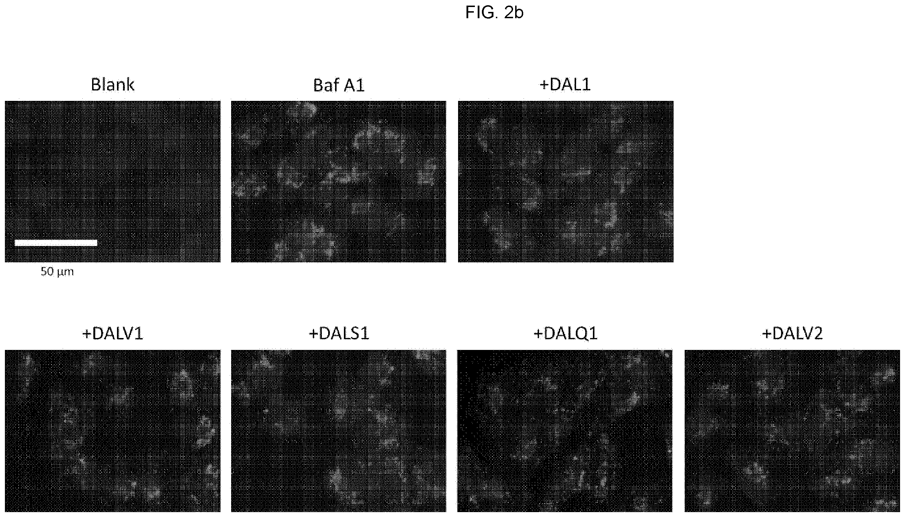

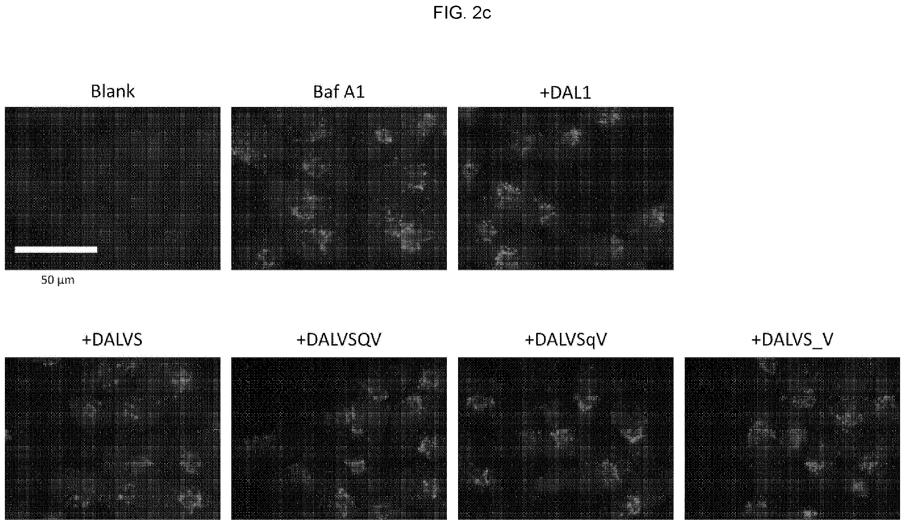

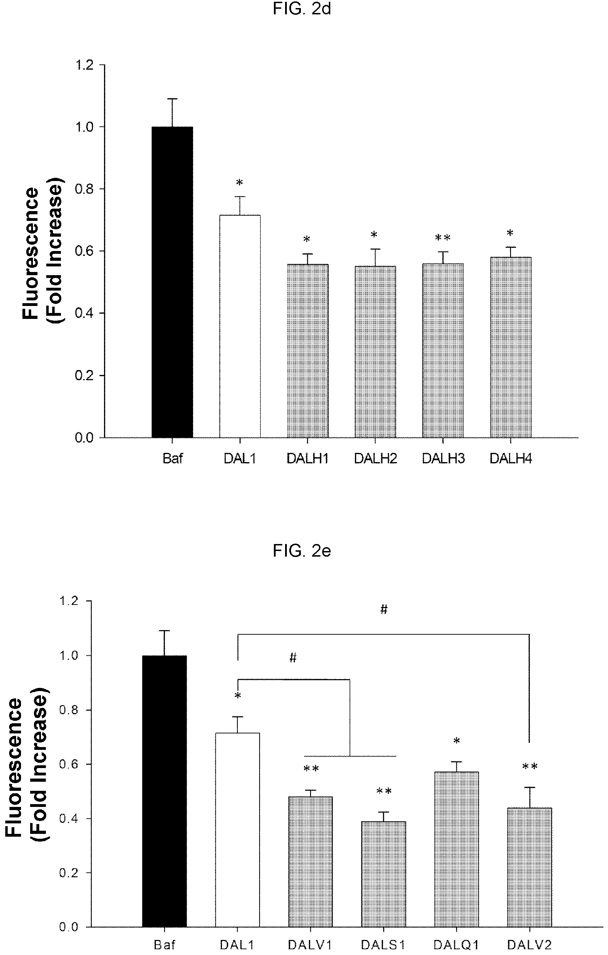

FIGS. 2a to 2c are series of fluorescence micrographs of LC3-GFP spots 12 h after treating H4 cell line (GL-H4) that stably overexpresses GFP-LC3 protein with various variant alloferon peptides according to one embodiment of the present invention in combination with bafilomycin at a concentration of 20 µM, showing that autophagic flux blocked by bafilomycin A-1 is restored by the variant alloferon peptides according to one embodiment of the present invention. FIGS. 2d to 2f are graphs showing quantification of the fluorescence intensity of LC3-GFP spots 12 h after treatment of the GL-H4 cell line with the variant alloferon peptides according to one embodiment of the present invention in combination with bafilomycin at a concentration of 20 µM. It can be seen that the spots are significantly increased after bafilomycin treatment due to the blocked autophagy, whereas the number and size of the spots decrease upon treatment with the variant alloferon peptides according to one embodiment of the present invention, which is a result of the resolution of autophagy. In FIGS. 2D to 2F, * indicates that the treatment of the peptides according to one embodiment of the present invention have a significant difference compared to the treatment with bafilomycin alone (* *p <* 0.05, *** p <* 0.01, **** p <* 0.001), and # indicates a significant difference compared to the treatment with bafilomycin and the previously reported D-type alloferon (DAL-1) (# p < 0.05).

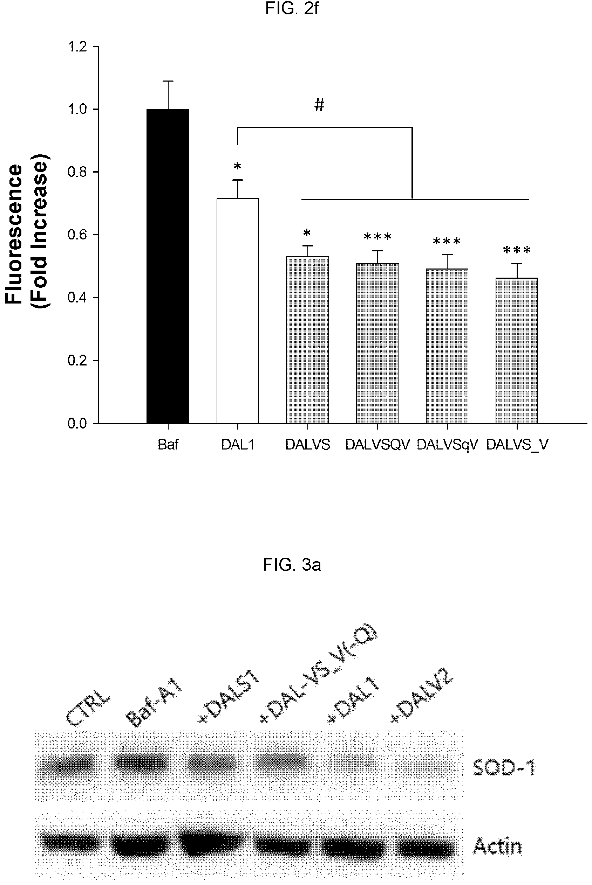

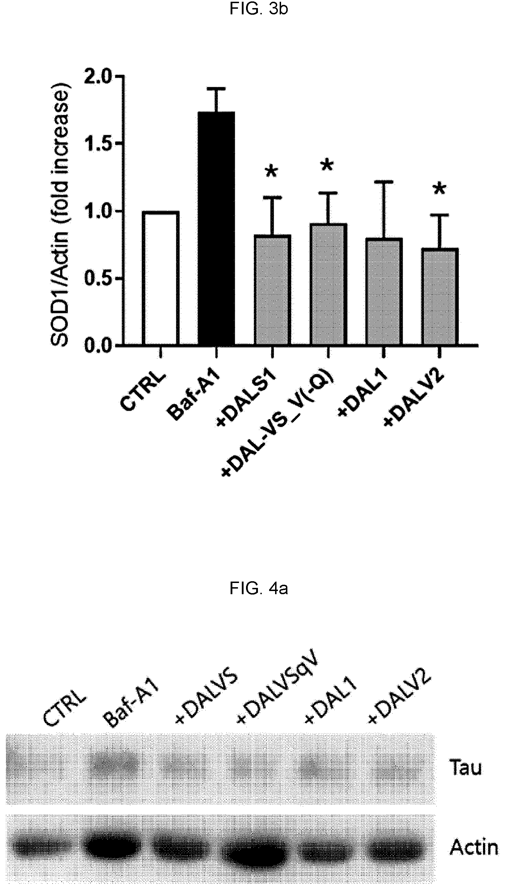

FIG. 3a is a result of Western blot analysis of the level of SOD1 protein expression in protein samples obtained 18 h after treatment of HEK293T cells transfected to express SOD1 with 100 nM bafilomycin A1 alone or in combination with 30 µM of some variant alloferon peptides according to one embodiment of the present invention, for investigating the degradation of SOD1 protein aggregates by treatment with some variant alloferon peptides (DALS1, DAL-VS_V(-Q), DAL1, and DALV2) according to one embodiment of the present invention. FIG.3b is a graph representing the results of quantifying relative expression of the SOD1 protein. In FIG.3b, * indicates a significant difference compared to the treatment with bafilomycin alone (** p* < 0.05).

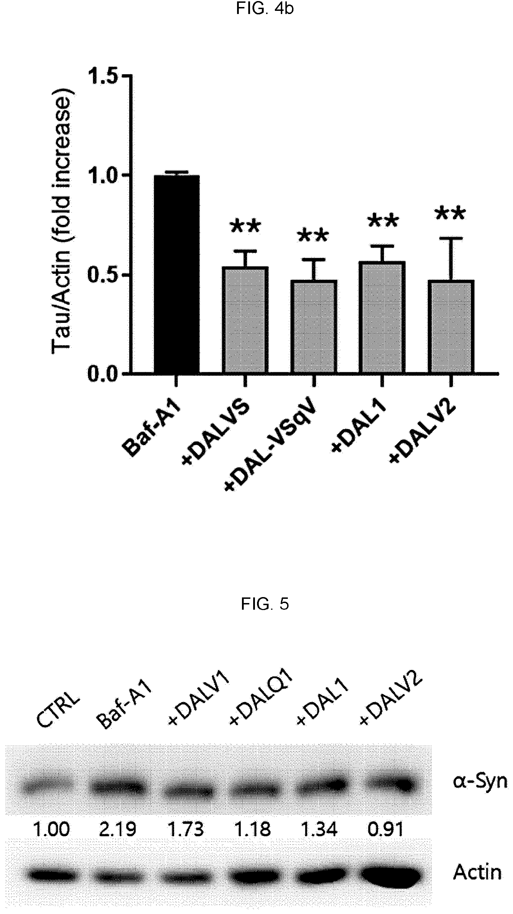

FIG. 4a is a result of Western blot analysis of the level of tau protein expression in protein samples obtained 18 h after treatment of HEK293T cells transfected to express tau protein with 100 nM bafilomycin A1 alone or in combination with 30 µM of some variant alloferon peptides according to one embodiment of the present invention, for investigating the degradation of tau protein aggregates by treatment with some variant alloferon peptides (DALS1, DAL-VS_V(-Q), DAL1, and DALV2) according to one embodiment of the present invention. FIG. 4b is a graph representing the results of quantifying relative expression of the tau protein. In FIG. 4b, * indicates a significant difference compared to the treatment with bafilomycin alone (** p* < 0.05).
FIG. 5 is a result of Western blot analysis of the level of α-synuclein protein expression in protein samples obtained 18 h after treatment of HEK293T cells transfected to express α-synuclein with 100 nM bafilomycin A1 alone or in combination with 30 µM of some variant alloferon peptides according to one embodiment of the present invention, for investigating the degradation of α-synuclein protein aggregates by treatment with some variant alloferon peptides (DALS1, DAL-VS_V(-Q), DAL1, and DALV2) according to one embodiment of the present invention.

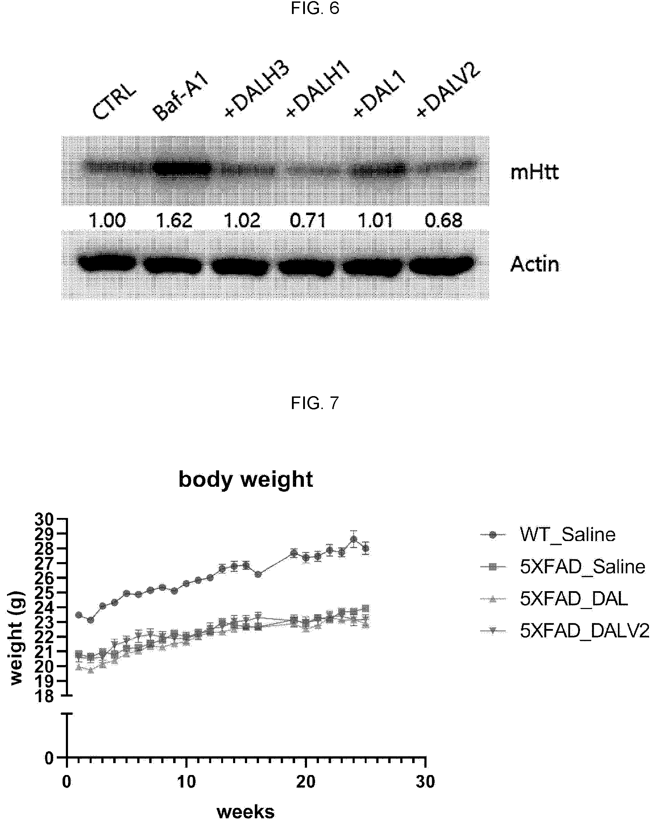

FIG. 6 is a result of Western blot analysis of the level of mutant huntingtin protein expression in protein samples obtained 18 h after treatment of HEK293T cells transfected to express mutant huntingtin protein with 100 nM bafilomycin A1 alone or in combination with 30 µM of some variant alloferon peptides according to one embodiment of the present invention, for investigating the degradation of mutant huntingtin protein aggregates by treatment with some variant alloferon peptides (DALS1, DAL-VS_V(-Q), DAL1, and DALV2) according to one embodiment of the present invention.
FIG.7 is a graph depicting the change in body weight of experimental animals upon administration of the variant alloferon peptides according to one embodiment of the present invention to Alzheimer-model animals for 25 weeks.

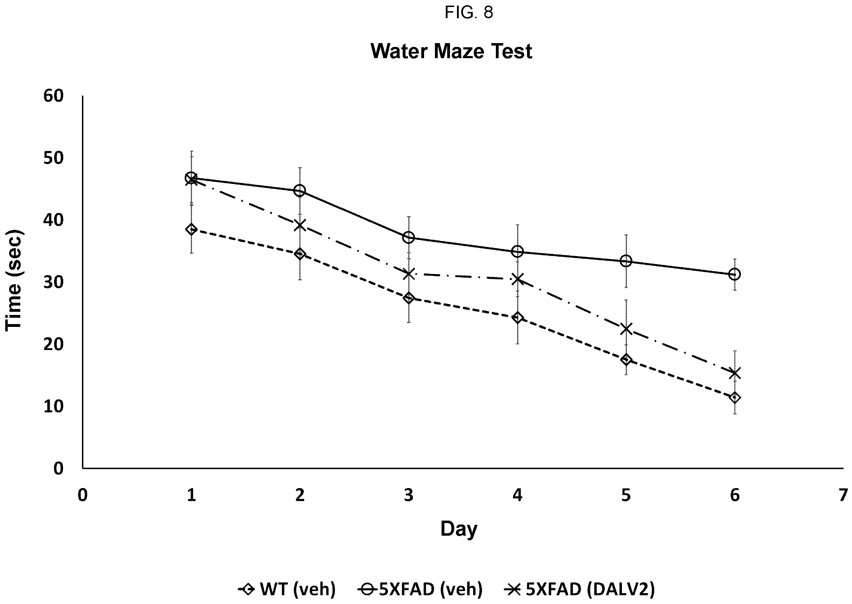

FIG.8 is a graph showing the ability of enhancing memory and learning of the variant alloferon peptides according to one embodiment of the present invention as determined by the Morris water maze test.

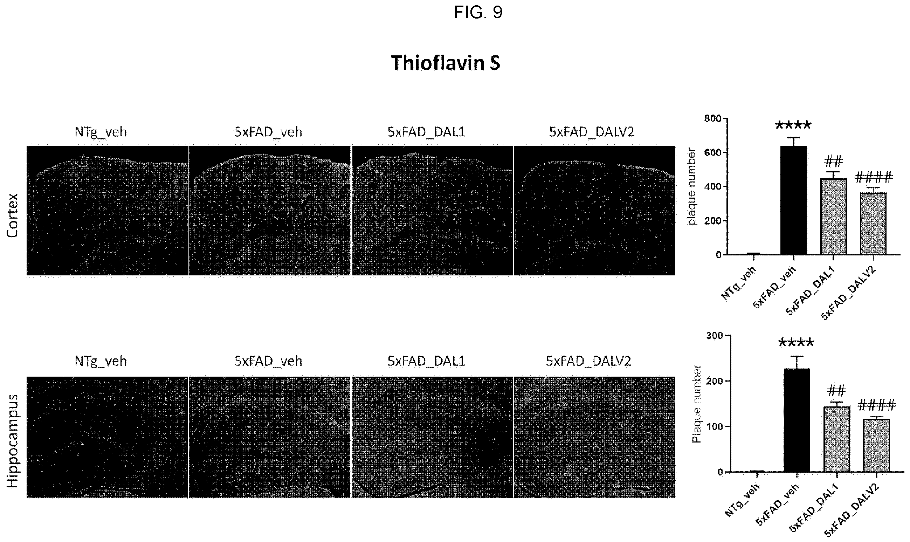

FIG.9 is a series of fluorescence micrographs (left) and a graph (right) quantifying the results of thioflavin S staining of β-amyloid aggregate plaques in cortical (top) and hippocampal (bottom) slices from experimental animals administrated with the variant alloferon peptides according to one embodiment of the present invention.

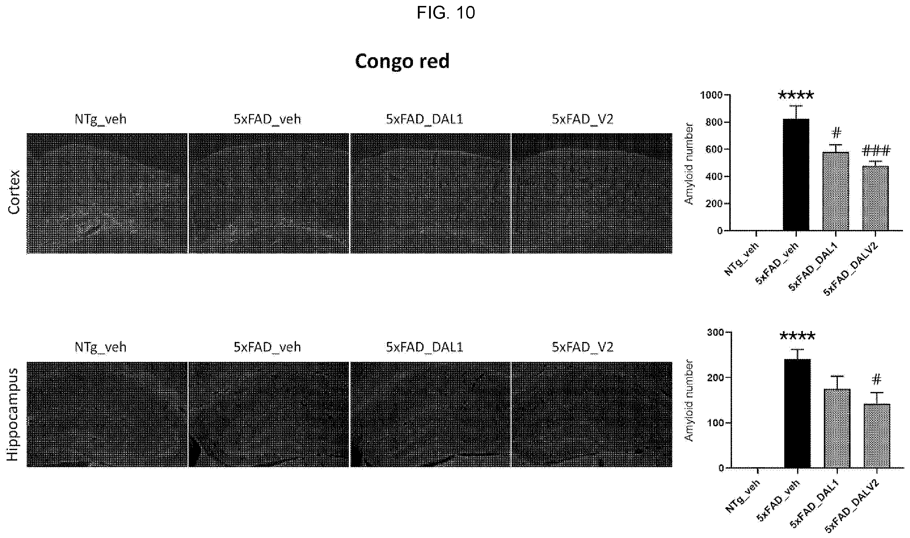

FIG.10 is a series of fluorescence micrographs (left) and a graph (right) showing the quantification of β-amyloid aggregate plaques stained with Congo red in cortical (top) and hippocampal (bottom) slices from experimental animals administrated with the variant alloferon peptides according to one embodiment of the present invention.

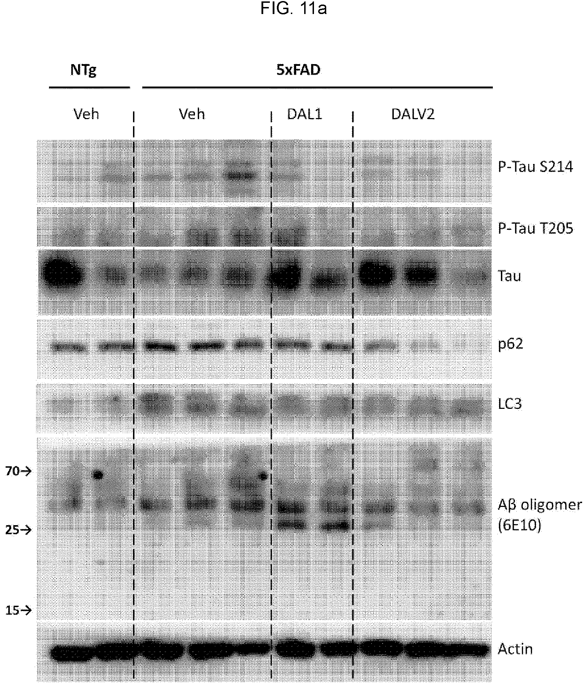

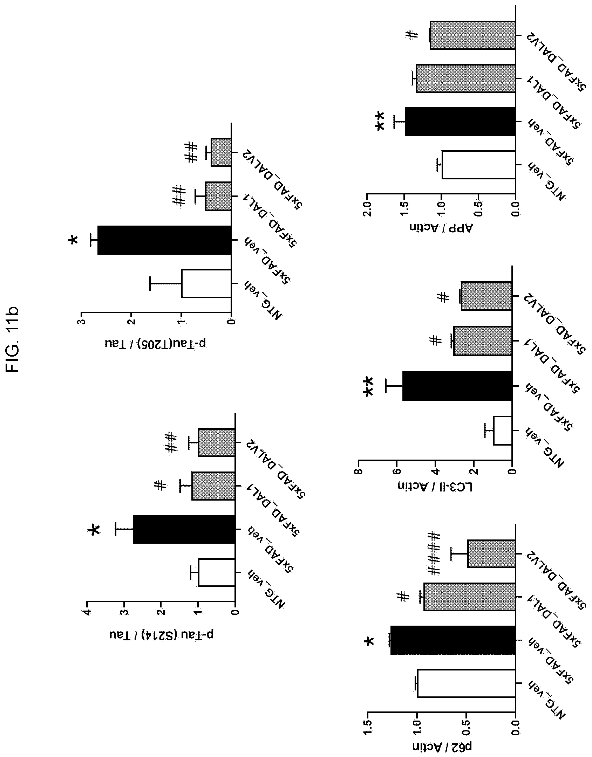

FIG.11A shows the results of a Western blot analysis to determine the expression of various pathogenic aggregate-forming proteins in the cerebral cortex of experimental animals administrated with the variant alloferon peptides according to one embodiment of the present invention, and FIG.11B is a series of graphs quantifying the results of the Western blot analysis.

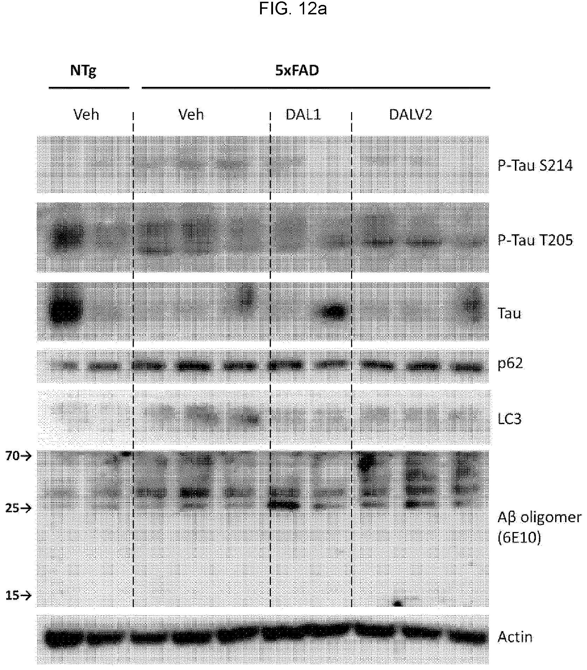

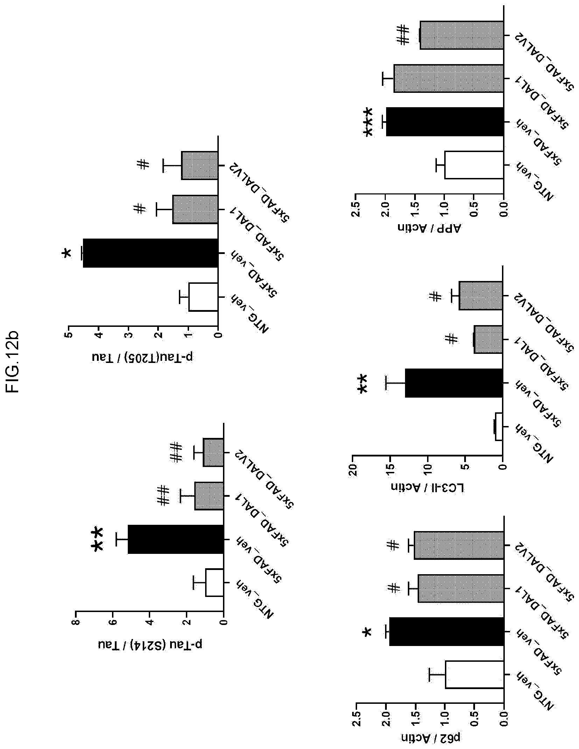

FIG.12A shows the results of a Western blot analysis to determine the expression of various pathogenic aggregate-forming proteins in the hippocampus of experimental animals administrated with the variant alloferon peptides according to one embodiment of the present invention, and FIG.12B is a series of graphs quantifying the results of the Western blot analysis.

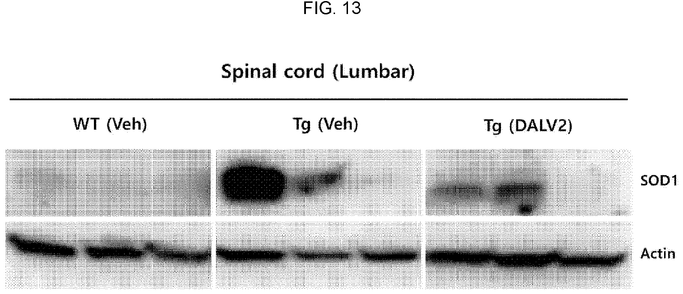

FIG.13 is a photograph depicting the results of a Western blot analysis on the spinal cord tissue of the lumbar spine obtained from ALS model animals sacrificed after administrating them with the variant alloferon peptides according to one embodiment of the present invention.

### BEST MODE FOR THE INVENTION

### Definitions of terms:

As used herein, the term "zinc homeostasis" refers to the mechanism that maintains a constant level of intracellular zinc, which are known to involve zinc transporters, zinc-binding proteins (metallothioneins, MTs), and transcription factors (MTF1-2).

As used herein, the term "Neurodegenerative disease" refers to a disease characterized by the progressive loss of structure or function of nerves due to the abnormal death of nerve cells. These neurodegenerative diseases include amyotrophic lateral sclerosis (ALS), Parkinson's disease (PD), Alzheimer's disease (AD), and Huntington's disease (HD).

As used herein, the term "abnormal or pathological protein aggregates" refers to abnormal aggregation of proteins, such as amyloid or tau, within cells to form insoluble fibrills. These abnormal or pathological protein aggregates are known to be a neuropathologic hallmark of various intermittent or inherited neurodegenerative diseases.

As used herein, the term "alloferon" refers to a natural peptide consisting of 13 amino acids isolated from the blood of larvae of bacterially infected *Calliphora vicina.* The alloferon is known as a non-toxic antiviral agent developed to treat infections caused by influenza virus, herpes virus, etc. (South Korean Patent No. 394864)

### Detailed descriptions of the invention:

In one aspect of the present invention, there is provided a novel peptide with a length of 12 to 20 a.a. capable of inhibiting the formation of abnormal protein aggregates, comprising an amino acid sequence designated by SEQ ID NO: 1 or 2, wherein 1 to 4 of the amino acids excluding glycine are L-type amino acids and the other amino acids are D-type amino acids.

The peptide may comprise an amino acid designated of SEQ ID NO: 1 or 2, wherein one, two, three or four amino acids excluding glycine are L-type amino acids and the other amino acids are D-type amino acids.

More specifically, if the peptide comprises the amino acid sequence designated as SEQ ID NO: 1, it may comprise at least one of the following:
i) at least one of the four histidines is a D-type amino acid;
ii) at least one of the third and eleventh amino acids, valine, is an L-type amino acid or both are D-type amino acids;
iii) the fourth amino acid, serine, is a D-type amino acid;
iv) the eighth amino acid, glutamine, is a D-type amino acid; and
v) at least two of i through iv above are D-type amino acids.

In the case that the peptide comprises an amino acid sequence designated as SEQ ID NO: 2, it may comprise at least one of the following:
i) at least one of the four histidines is a D-type amino acid:
ii) at least one of the third and tenth amino acids, valine, is an L-type amino acid, or both are D-type amino acids;
iii) all the fourth amino acid, serine, is a D-type amino acid; and
iv) at least two of i to iii are D-type amino acids.

According to a preferred embodiment, the peptide of the present invention is an L-type alloferon peptide of SEQ ID NO:1 in which at least three of the four histidines are substituted with D-type histidines, also, at least two of the following amino acids are L-or D-type amino acids or are deleted: two valines, serine the third amino acid, and glutamine the eighth amino acid. Preferably, the peptide according to one embodiment of the present invention may comprise the amino acid sequence of any one of SEQ ID NOs: 2 to 14, and more preferably the amino acid sequence of any one of SEQ ID NOs: 3 to 5, 7, 8, 10, and 12 to 14.

As shown in FIG.1, the present inventors have found that L-type alloferon, which is naturally present in the body, exhibits excellent activity under *in vitro* conditions, but has an extremely short half-life (T_{1/2} = 51.77 min) when administered *in vivo,* which makes it difficult to be developed into a practical medicine. Therefore, based on the fact that the alloferon peptide of the present invention not only exhibits bioactivity by interacting with certain proteins *in vivo* but also functions as a zinc-binding protein and thus acts as a zinc homeostasis maintainer by transporting zinc from outside the cell into the cell, the present inventors hypothesized that a D-type peptide, which is not naturally present *in vivo,* could substitute for the L-type peptide, which is readily degraded *in vivo.* The present inventors synthesized the D-type alloferon and performed the same experiment as that using the L-type alloferon, and confirmed that the D-type alloferon exhibits the same biological activity as the L-type alloferon. Moreover, as a result of further research on whether the D-type alloferon in which some amino acids are substituted with those of the L-type would show similar functionality, it was found that substituting either one of the histidines expected to function as zinc chelators, or valine the third amino acid and/or serine the fourth amino acid, or, glutamine the eighth amino acid and/or valine the eleventh amino acid in the D-type alloferons with an L-type amino acid respectively, results in biological activity that is equivalent or even superior to the D-type alloferon. Furthermore, the best biological activity was found for the variant with the removal of glutamine, the eighth amino acid.

In another aspect of the present invention, there is provided a pharmaceutical composition for the treatment of neurodegenerative diseases comprising the peptide above as an active ingredient.

In the pharmaceutical composition, the neurodegenerative disease may be a neurodegenerative disease having the formation of abnormal protein aggregates as its etiology or pathology, wherein the abnormal protein aggregates are formed by the abnormal aggregation of α-synuclein, β-amyloid, TDP-34, p62, FUS protein, superoxide dismutase-1 (SOD-1), huntingtin protein, or tau protein. The neurodegenerative disease may specifically be Alzheimer's disease (AD), which is associated with the accumulation of β -amyloid protein or tau protein aggregates; Parkinson's disease (PD), which is associated with the accumulation of α-synuclein aggregates; Amyotrophic lateral sclerosis (ALS), which is associated with the accumulation of SOD-1 aggregates; Huntington's disease (HD), which is associated with the accumulation of Huntingtin protein aggregates; chronic traumatic encephalopathy, which is associated with the accumulation of aggregates of tau protein or TAR DNA-binding protein 43 (TDP-43); Lytico-bodig disease, which is associated with the accumulation of tau protein aggregates; fronto-temporal lobe degeneration, which is associated with the accumulation of tau protein, TAR DNA-binding protein 43 (TDP-43), fused in sarcoma (FUS) protein, or p62 protein aggregates; corticobasal degeneration, which is associated with the accumulation of tau protein aggregates; progressive supranuclear palsy, which is associated with the accumulation of tau protein aggregates. In addition, diseases such as Creuzfeldt-Jakob disease, Gerstmann-Straussler-Scheinker syndrome, fatal familial insomnia, meningioangiomatosis, and neuronal ceroid lipofuscinoses are known to be associated with the accumulation of protein aggregates in nerves. These neurodegenerative diseases and their association with the accumulation of protein aggregates in nerves are well described in the prior art (Strømland et al., J. Clin. Transl. Res. 2(1): 11-26, 2016; Tutar et al., Neurodegenerative Diseases, published: May 15th, 2013, DOI: 10.5772/54487; Diez-Ardanuy et al., Sci. Rep: 7(1): 10, 2017).

**In** the pharmaceutical composition, the peptide can treat the neurodegenerative diseases by preventing neuronal cell death, regulating intracellular zinc homeostasis, and promoting lysosomal function.

The pharmaceutical composition according to one embodiment of the present invention may comprise a pharmaceutically acceptable carrier, and may further comprise pharmaceutically acceptable adjuvants, excipients or diluents in addition to the carrier.

As used herein, the term "pharmaceutically acceptable" refers to a composition that is physiologically acceptable and does not normally cause allergic reactions such as gastrointestinal disturbances, dizziness, or similar reactions when administered to humans. Examples of such carriers, excipients, and diluents include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oil. Additionally, fillers, anti-flocculants, lubricants, wetting agents, flavors, emulsifiers, and preservatives may be further included.

Furthermore, the pharmaceutical composition according to one embodiment of the present invention can be formulated using methods known in the art to mediate rapid release, or sustained or delayed release of the active ingredients upon administration to a mammal. Formulations include powders, granules, tablets, emulsions, syrups, aerosols, soft or hard gelatin capsules, sterile injectable solutions, and sterile powder forms.

The pharmaceutical composition according to one embodiment of the present invention may be administered by various routes, for example, orally, or parenterally, e.g., as a suppository, transdermally, intravenously, intraperitoneally, intramuscularly, locally at the site of the lesion, intranasally, or intrathecally. It may also be administered by using an implant device for sustained, continuous, or repeated release. The frequency of administration may be once or multiple times per day within any desired range, and the duration of administration is not specifically limited.

The pharmaceutical composition according to one embodiment of the invention may be administered by conventional systemic administration or localized administration, for example by intramuscular or intravenous injection. Furthermore, the peptides according to one embodiment of the invention can also be administered orally.

The pharmaceutical composition according to one embodiment of the present invention can be formulated in any suitable form with any commonly used pharmaceutically acceptable carrier. Pharmaceutically acceptable carriers include, for example, water, suitable oils, saline, carriers for parenteral administration such as aqueous glucose and glycols, and may further include stabilizers and preservatives. Suitable stabilizers include antioxidants such as sodium hydrogen sulfite, sodium sulfite, or ascorbic acid. Suitable preservatives include benzalkonium chloride, methyl- or propyl-paraben, and chlorobutanol. The pharmaceutical composition according to the present invention may also comprise suspending agents, solubilizing agents, stabilizing agents, isotonic agents, preservatives, anti-adsorbents, surfactants, diluents, excipients, pH adjusters, painless reliever, buffering agents, and antioxidants, depending on the method of administration or formulation. Pharmaceutically acceptable carriers and formulations suitable for the present invention, including those exemplified above, are described in detail in the literature [Remington's Pharmaceutical Sciences, latest edition]

The dosage of the pharmaceutical composition according to one embodiment of the present invention to a patient depends on many factors, including the patient's height, body surface area, age, the specific compound being administered, sex, time and route of administration, general health, and other medications being administered concurrently. The therapeutically active alloferon or polynucleotide encoding it may be administered in an amount of 100 ng to 1000 mg per body weight (kg), more preferably in an amount of 1 µg to 100 mg per body weight (kg), and most preferably in an amount of 5 to 40 mg per body weight (kg), while the dose may be adjusted taking into account the above factors.

Furthermore, the pharmaceutical composition of the present invention is administered in a therapeutically effective amount.

As used herein, the term "therapeutically effective amount" refers to an amount sufficient to treat a condition with a reasonable benefit/risk ratio applicable to medical treatment, and the effective dose level may be determined based on factors including the type of individual, severity, age, sex, activity of the drug, sensitivity to the drug, time of administration, route of administration and excretion level, duration of treatment, concomitant medications, and other factors well known in the medical field. The pharmaceutical composition of the present invention may be administered at a dose of 0.1 mg/kg to 1 g/kg, more preferably at a dose of 1 mg/kg to 500 mg/kg. Meanwhile, the dosage may be appropriately adjusted according to the age, sex and condition of the patient.

In another aspect of the present invention, there is provided a method of treating a subject suffering from a neurodegenerative disease, comprising administering a therapeutically effective amount of the peptide to the subject.

In another aspect of the present invention, there is provided a method of inhibiting the accumulation of pathogenic protein aggregates in the nervous system of a subject suffering from a neurodegenerative disease, comprising administering a therapeutically effective amount of the peptide to the individual.

In any of the above methods, the composition may be administered by oral or parenteral administration as described above, and in the case of parenteral administration, it may be administered by either systemic or topical administration. In the case of systemic administration, it may be administered by intravenous injection, intraperitoneal injection, or intramuscular injection. On the other hand, in the case of topical administration, it may be administered by intracranial administration, intracerebrospinal administration, or subcutaneous injection.

As used herein, the term "therapeutically effective amount" means an amount that produces a palliative, inhibitory, ameliorative, and/or curative effect on the symptoms of the condition being treated.

### MODE FOR THE INVENTION

The present invention will now be described in more detail with reference to the following examples. However, the present invention is not limited to the embodiments disclosed herein, but may be embodied in many different forms, and the following embodiments are provided to make the disclosure of the invention complete and to give those of ordinary skill in the art a complete idea of the scope of the invention.

### Example 1: Preparation of peptides

### 1-1: Synthesis of L-Type Peptide

The L-type peptide (SEQ ID NO: 16, LAL) of alloferon (SEQ ID NO: 1) used in the present invention was synthesized by Corp. Peptron (Korea).

### 1-2: Synthesis of D-Type Peptide

The D-type alloferon peptide (SEQ ID NO: 15, DAL1), in which all amino acids except glycine have been substituted with D-type amino acids instead of the L-type alloferon used in the present invention, was also synthesized by Corp. Peptron (Korea) and Anigen (Korea).

### Experimental example 1: In vitro plasma stability assay

As the present inventors believed that the mechanism of action of alloferon peptides is through endocytosis via multi-ligand receptors rather than physiological activity and that zinc-binding capacity is an important mechanism, they investigated the blood stability of the D-type alloferon of Example 1-2 in which all amino acids except glycine have been replaced with D-type amino acids and the L-type alloferon of Example 1-1. An *in vitro* plasma stability assay was performed to compare the stability of the L-type peptide and the D-type peptide of alloferon in the plasma of mice. For this purpose, 500 to 600 µl of blood was obtained from ICR mice by orbital blood sampling, which was subsequently centrifuged at 2000xg for 20 min at 4°C using a centrifuge, and the supernatant was collected to obtain plasma. After adding 10 µM of the alloferon L-type and D-type peptides to 100 µl of the plasma, the plasma was incubated at 37°C for 0, 5, 10, 30, 60, and 90 min. Then, 500 µl of cold methanol was added to precipitate the protein, and the protein was lyophilized and subjected to liquid chromatography and protein mass spectrometry. The results showed that the alloferon L-type peptide was degraded early in plasma, while the D-type peptide remained stable over time without degradation (FIG. 1).

### 1-3: Design and synthesis of variant alloferon peptides

From the results of Experimental example 1 described above, the present inventors investigated whether a variant alloferon peptide in the form of an L-type/D-type hybrid where some amino acids are L-type amino acids, instead of the D-type peptide (DAL1) where all amino acids except glycine have been substituted with D-type amino acids, would exhibit equivalent biological activity. To this end, 12 variant alloferon peptides in which one to four amino acids are L-type amino acids were synthesized (Table 1).

**[Table 1]**

| Peptides according to one embodiment of the present invention | | | | | | | | | | | | | | |
|---|---|---|---|---|---|---|---|---|---|---|---|---|---|---|
| Peptide name | Amino acid sequences | | | | | | | | | | | | | SEQ ID NOs |
| Alloferon | H* | G | V* | S* | G | H* | G | Q* | H* | G | V* | H* | G | 1 |
| Alloferon ΔQ | H* | G | V* | S* | G | H* | G | -. | H* | G | V* | H* | G | 2 |
| DALVS | h | G | V | S | G | h | G | q | h | G | v | h | G | 3 |
| DALV2 | h | G | v | s | G | h | G | q | h | G | V | h | G | 4 |
| DALS1 | h | G | v | S | G | h | G | q | h | G | v | h | G | 5 |
| DALH3 | h | G | v | s | G | h | G | q | H | G | v | h | G | 6 |
| DALV1 | h | G | V | s | G | h | G | q | h | G | v | h | G | 7 |
| DALH1 | H | G | v | s | G | h | G | q | h | G | v | h | G | 8 |
| DALH2 | h | G | v | s | G | H | G | q | h | G | v | h | G | 9 |
| DALQ1 | h | G | v | s | G | h | G | Q | h | G | v | h | G | 10 |
| DALH4 | h | G | v | s | G | h | G | q | h | G | v | H | G | 11 |
| DALVSQV | h | G | V | S | G | h | G | Q | h | G | V | h | G | 12 |
| DALVSqV | h | G | V | S | G | h | G | q | h | G | V | h | G | 13 |
| DALVS_V(ΔQ) | h | G | V | S | G | h | G | -. | h | G | V | h | G | 14 |
| DAL1 (D-alloferon) | h | G | v | s | G | h | G | q | h | G | v | h | G | 15 |
| LAL (L-alloferon) | H | G | V | S | G | H | G | Q | H | G | V | H | G | 16 |
| Capitalized letters indicate L-type amino acids, lowercase letters indicate D-type amino acids, and the capitalized letters with * indicates that the amino acid can be either L- or D-type. | | | | | | | | | | | | | | |

The variant peptide comprises the products of reverting one of four histidines that are thought to be involved in zinc chelation to L-type (DALH1, DALH2, DALH3, DALH4), the products of reverting at least one of the third, fourth and eleventh amino acids to an L-type amino acid (DALVS, DALV2, DALV1, and DALS1), the products of reverting glutamine, the eighth amino acid, to an L-type amino acid (DALQ1), and the products of reverting at least three amino acids among two valines, glutamine and serine, to L-type amino acids (DALVSQV, DALVSqV, and DALVS_V).

The synthesis of the peptides was performed by Corp. Peptron (Korea) and Anigen (Korea).

### Experimental Example 2: Cell Culture

H4 cell line (GL-H4) and HEK293T cell line permanently transfected with GFP-LC3 plasmid were used in the present invention. The cell culture medium was Minimum Essential Medium (MEM, WellGene) supplemented with 10% fetal bovine serum (Hyclone, USA) and antibiotic and antifungal mixture (WellGene, Korea) and cultured in a cell culture machine at 37°C and 5% CO₂.

### Experimental Example 3: Analysis of Autophagic Flow Blocked by Bafilomycin A-1

To investigate the effect of peptides comprising the amino acid sequences designated as SEQ ID NO: 3 to 16 on autophagic flow, the present inventors treated H4 cell lines (GL-H4) expressing GFP-linked LC3 with the peptides (20 µM) and/or the autophagic inhibitor Bafilomycin A-1 (Baf A1, 100 nM) to inhibit autophagy, and then the effect of the variant alloferon peptides according to one embodiment of the present invention was investigated by fluorescence microscopy analysis.

As a result, the LC3 spots shown in FIGS. 2a to 2c are caused by the LC3-GFP protein present in autophagosomes, which, if not degraded by the lysosome, inhibits the entire autophagy process, indicating the accumulation of autophagosomes. When the GL-H4 cell line was treated with 100 nM of bafilomycin (Baf A1) to inhibit autophagic flow, there was a significant increase in the number and size of LC3 spots, which was reduced upon treatment with a variant alloferon peptide (20 µM) according to one embodiment of the present invention (FIGS. 2a to 2c). When quantified by measuring the intensity of fluorescence of the whole spot, the intensity of fluorescence was similarly reduced upon treatment with a variant alloferon peptide according to one embodiment of the present invention (FIG.s 2d to 2f). This indicates that autophagosomes accumulated due to blocked autophagy are scavenged by the variant alloferon peptides according to one embodiment of the present invention. In particular, as shown in FIGS. 2e and 2f, among the variant alloferon peptides according to one embodiment of the present invention, those produced by reverting at least one to three amino acids among two valines, serine and glutamine to L-type amino acids (DALV1, DALS1, DALV2, DALVS, and DALVSq), or, reverting all amino acids except histidine to an L-type (DALVSQV), and those produced by removing glutamine from the DALVSQV (DALVS_V), exhibited superior autophagic scavenging activity compared to D-type alloferon peptide (DAL1), in which all amino acids have been replaced with D-type amino acids. Autophagic killing activity was found to be best when at least three of the four amino acids were L-type amino acids.

### Experimental Example 4: Analysis of the effect of variant alloferon peptides on protein aggregate formation following SOD1 protein overexpression

The present inventors then investigated whether some of the variant peptides (DALS1, DALVS_V, DAL1, DALV2) comprising the amino acid sequences designated as SEQ ID NO: 3 through 16 could resolve SOD1 aggregates that formed after transient transfection of SOD1 protein into the 293T cell line. To this end, EGFP-SOD1 G93A DNA was transiently transfected into the HEK293T cell line using Lipofectamine 2000. After transfection, the cell lines were incubated in a cell culture machine at 37°C and 5% CO₂ for 30 h, and the culture medium was replaced with Minimum Essential Medium (MEM, Gibco, USA). Subsequently, the cells were pretreated with 100 nM bafilomycin for 30 min, which were then treated with µM variant alloferon peptide and incubated for 18 h. After that, proteinase inhibitors and phosphatase inhibitors (2 µg/ml aprotinin, 2 µg/ml leupeptin, 1 µg/ml pepstatin A, 1 mM phenyl-methylsulfonyl fluoride (PMSF), 1 mM Na₃ VO₄, 5 mM NaF and 10 mM Na₄P₂O₇) were put into a 6-well culture dish at 200 µl per well to lyse the cells. The obtained cell lysate was first left at 30 min for 4°C. Then, it was subjected to protein quantification using BCA protein assay kit (Pierce Biotechnology, USA), and centrifuged at 17,000xg for 20 min, where the supernatant was discarded and only the cell debris was taken to obtain protein. Subsequently, 5X sample buffer (300 mM Tris, pH 6.8, 10% SDS, 50% Glycerol, 0.1% BPB, 2.5% Mercaptoethanol, 100 mM DTT) was mixed with the quantified sample, denatured at 95 °C for 5 min, and prepared for analysis. Then, electrophoresis was performed using 8% to 15% SDS-polyacrylamide gel, and the proteins separated by their sizes were transferred to polyvinylidene difluoride (PVDF) membranes (Millipore, USA). The protein-transferred membranes were then blocked with 3% skim milk powder in TBST for 1 h. The blocked membranes were reacted with anti-GFP antibody (Santa Cruz biotechnology, USA), while anti-Actin antibody (Sigma, USA) was used as a loading control. The antibodies, each recognizing a specific protein, were added to a solution of 1% BSA dissolved in TBST. For all blots, secondary antibodies were diluted at a ratio of 1:10,000 in 2% skim milk and incubated. Protein signals were detected using enhanced chemiluminescence (iNTRoN Biotechnology, South Korea) and a bio-imaging system (MF-Chemibis, Shimadzu Scientific Korea Corporation, South Korea).

As a result, as shown in FIG.3a, it can be seen that SOD1 protein aggregates have been increased by bafilomycin compared to the control. Here, the protein aggregates were reduced by some variant alloferon peptides according to one embodiment of the present invention, and quantification also confirmed the resolution of the aggregate accumulation by some variant alloferon peptides according to one embodiment of the present invention. In particular, as shown in FIG.3b, among the variant alloferon peptides according to one embodiment of the present invention, those produced by reverting the fourth and/or eleventh amino acid to an L-type amino acid (DALS 1, DALV2), those produced by reverting at least three among two valines, glutamine, and serine to an L-type amino acid (DALVS_V), exhibited better protein aggregate dissociating ability compared to the D-type alloferon peptide in which all amino acids have been substituted with D-type amino acids (DAL1).

### Experimental example 5: Analysis of the effect of variant alloferon peptides on protein aggregate formation after mutant tau protein overexpression

The present inventors then investigated whether some of the variant peptides (DALVS, DALVSqV, DAL1, and DALV2) comprising the amino acid sequences designated as SEQ ID NO: 3 through 16 could resolve the mutant tau protein aggregates that formed after transient transfection of the mutant tau protein into the HEK293T cell line. For this purpose, EGFP-Tau P301L DNA was transiently transfected into the HEK293T cell line using Lipofectamine 2000. Protein aggregate analysis was performed in the same manner as in Experimental example 4 above.

As a result, as shown in FIG.4a, it can be seen that mutant tau protein aggregates have been increased by bafilomycin compared to control. Quantification also confirmed that protein aggregates were reduced by some variant alloferon peptides according to one embodiment of the present invention, as shown in FIG.4b.

### Experimental example 6: Analysis of the effect of variant alloferon peptides on protein aggregate formation after α-synuclein protein overexpression

The present inventors then investigated whether some of the variant peptides (DALV1, DALQ1, DAL1, DALV2) comprising the amino acid sequences designated as SEQ ID NO: 3 through 16 could resolve the α-synuclein protein aggregates that formed after transient transfection of α-synuclein protein into the HEK293T cell line. For this purpose, EGFP-α-synuclein A53T DNA was transiently transfected into the HEK293T cell line using Lipofectamine 2000. Protein aggregate analysis was performed in the same manner as in Experimental example 4 above.

As a result, it can be seen in FIG.5 that α-synuclein protein aggregates have been increased by bafilomycin compared to the control. It was also confirmed that protein aggregates were reduced by some variant alloferon peptides according to one embodiment of the present invention. In this case, DALQ1 and DALV2 also showed better protein aggregate dissociating ability compared to the D-type alloferon peptide (DAL-1) in which all amino acids have been substituted with D-type amino acids.

### Experimental example 7: Analysis of the effect of variant alloferon peptides on protein aggregate formation after overexpression of mutant huntingtin protein

The present inventors then investigated whether some of the variant peptides (DALH3, DALH1, DAL1, and DALV2) comprising the amino acid sequences designated as SEQ ID NO: 3 through 16 could resolve the mutant huntingtin protein aggregates that formed after transient transfection of the mutant huntingtin protein into HEK293T cell lines. For this purpose, GFP-mHttQ74 DNA was transiently transfected into the HEK293T cell line using Lipofectamine 2000. Protein aggregate analysis was performed in the same manner as in Experimental example 4 above.

As a result, it can be seen in FIG.6 that mutant huntingtin protein aggregates were increased by bafilomycin compared to the control, and that protein aggregates were reduced by some of the variant alloferon peptides according to one embodiment of the present invention. In this case, DALH1 and DALV2 also showed better protein aggregate dissociating ability compared to the D-type alloferon peptide (DAL1) in which all amino acids have been substituted with D-type amino acids.

### Experimental example 8: Animal experiments for Alzheimer's disease 8-1: Body weight changes following administration of variant alloferon peptides

The present inventors conducted animal experiments using disease model animals to determine whether the variant alloferon peptides from the results of Experimental experiments 4 through 7 above according to one embodiment of the present invention can be used as therapeutics for various neurodegenerative diseases.

First, to determine whether it is toxic when administered *in vivo,* a dementia model animal (5XFAD) was administrated with 20 mg/kg of a variant alloferon peptide (DALV2) according to one embodiment of the present invention by intraperitoneal (IP) injection once daily, five days a week (Monday through Friday) for 25 weeks to investigate changes in body weight. DAL in which all amino acids are D-type was used as a control.

As a result, as shown in FIG. 7, weight gain was consistent over the 25-week injection period, with no differences between treatment groups.

### 8-2: Morris water maze test

Because the present inventors observed from the results of Experimental examples 4 through 7 above that the variant alloferon peptides according to one embodiment of the present invention breaks down abnormal protein aggregates, they intraperitoneally administered the variant alloferon peptides according to one embodiment of the present invention to Alzheimer's model mice (5XFAD) at a concentration of 20 mg/kg once a day, 5 days a week (Monday through Friday) for 25 weeks, and observed whether the memory of the experimental animals improved from day 1 to day 6 after 25 weeks of administration by Morris water maze test.

As a result, as shown in FIG.8, the peptide according to one embodiment of the present invention significantly restored memory and learning ability in Alzheimer's model animals.

### 8-3: Analyze for inhibition of β-amyloid aggregate accumulation in brain tissue

To determine whether the improvement in the behavioral indices observed in Experimental example 8-2 above resulted from the inhibition of β-amyloid aggregate accumulation in the brain, the present inventors obtained brain tissue sections after sacrificing the animals that completed the Morris water maze test shown in FIG.8. These sections were stained using thioflavin S, which has a selective affinity for β-amyloid plaques, in order to visualize the β-amyloid aggregates.

Specifically, for thioflavin S staining, the brain of a dementia model mouse was harvested and freeze-dried in O.C.T compound (Sakura finetec, USA), and brain sections were obtained by coronal sectioning and mounted on glass slides coated with 0.1% poly-L-lysine. The obtained brain sections were washed in 70% ethanol for 1 min, then in 80% ethanol for 1 min again, and stained in 1% thiflavin S solution (MilliporeSigma, USA) for 15 min. After that, it was sequentially washed in 80% ethanol for 1 min, in 70% ethanol for 1 min again, and then washed twice in distilled water. Finally, the β-amyloid senile spots were observed through fluorescence microscopy.

Congo red staining for identification of amyloid was performed as follows. The brain sections obtained by coronal sectioning were mounted on glass slides coated with 0.1% poly-L-lysine. The obtained brain sections were washed in Phosphate-buffered Saline (PBS) solution for 2 min and then stained in 0.5% Congo red solution (Thermo fisher, USA) for 20 min. After washing the Congo red solution in PBS solution, the sections were dipped in and out of alkaline alcohol solution containing 0.01% sodium hydroxide 10 times rapidly. The sections were sequentially washed in PBS solution for 3 min, 95% ethanol for 3 min, and then 100% ethanol for 3 min for dehydration. Finally, the amyloid was observed under a fluorescence microscope, and the number of stained senile spots and amyloid per brain section was counted and quantified with a statistical program (Graph prism).

As a result, as shown in FIGS. 9 and 10, in Alzheimer's model animals, a significant number of β-amyloid plaques were identified, but neither the control DAL1 nor the variant alloferon peptides according to one embodiment of the present invention significantly inhibited the accumulation of β-amyloid aggregates.

### 8-4: Analyis of the Effects on the Expression of Different Aggregate-forming Proteins in the Brain

The present inventors conducted a western blot analysis to investigate the expression of pathogenic aggregate-forming proteins in brain tissue, such as tau protein, p62, and LC3, other than β-amyloid from the results of Experimental examples 8-2 and 8-3 above.

Specifically, cortical and hippocampal tissues were obtained from experimental animals sacrificed after the behavioral experiments, and the tissues were lysed and subjected to Western blot analysis using antibodies that specifically bind to phosphorylated tau S214 (P-Tau S214), phosphorylated tau T205 (P-Tau T205), unphosphorylated tau, p62, LC3, and β-amyloid (APP), respectively.

As a result, as shown in FIGS. 11A to 12B, all six proteins were increased in the cerebral cortex as well as in the hippocampus in the Alzheimer's disease model mice compared to normal mice, which were the control, whereas animals treated with either the control DAL1 or the peptide (DALV2) according to one embodiment of the present invention showed significantly lower expression of the above aggregate-forming proteins in the cortex and hippocampus. In cortical Western blot analysis, DALV2 showed better protein aggregate dissociating ability compared to the D-type alloferon peptide (DAL1) in which all amino acids were substituted with D-type amino acids. Western blot analysis of hippocampal sections showed that DALV2 significantly reduced the amount of β-amyloid, whereas DAL1 did not show significance in reducing the β-amyloid level. These results demonstrate that the peptides according to one embodiment of the present invention can inhibit the formation of pathogenic protein aggregates in the brain after *in vivo* administration, with superior inhibition of DALV2.

### Experimental example 9: Analysis of the effects on amyotrophic lateral sclerosis model animals

To determine whether the variant alloferon peptides according to one embodiment of the present invention have a therapeutic effect on amyotrophic lateral sclerosis (ALS) *in vivo,* the present inventors administered a peptide according to one embodiment of the present invention (DALV2) to amyotrophic lateral sclerosis model animals (SOD1G93A) at an age of 112 to 180 days. The peptide was administered at a dose of 40 mg/kg by subcutaneous (SC) injection, repeated five days a week (Monday through Friday). After the experiment, the animals were sacrificed and spinal cord slices from the lumbar region were obtained and subjected to Western blot analysis using anti-SOD-1 antibody.

As a result, as shown in FIG. 13, significantly lower levels of SOD-1 aggregates were detected in ALS model animals treated with the variant alloferon peptides according to one embodiment of the present invention. This demonstrates that the variant alloferon peptides according to one embodiment of the present invention inhibit the accumulation of pathogenic protein aggregates in the nervous system, even *in vivo.*

The present invention has been described with reference to the embodiments described above, but these are exemplary only, and one having ordinary skill in the art will understand that various modifications and other equally valid embodiments are possible from them. The true scope of technical protection of the invention should therefore be determined by the technical ideas of the appended claims of the patent.

### INDUSTRIAL APPLICABILY

The peptides according to one embodiment of the present invention can be developed as therapeutic agents for various neurodegenerative diseases, such as Alzheimer's disease and amyotrophic lateral sclerosis, caused by pathogenic protein aggregates, by inhibiting abnormal hyperphosphorylation of pathogenic proteins and the resulting aggregate formation in the central nervous system, and removing the parasitic protein aggregates.

## Claims

1. A novel peptide with a length of 12 to 20 a.a. capable of inhibiting the formation of abnormal protein aggregates, comprising an amino acid sequence designated by SEQ ID NO: 1 or 2, wherein 1 to 4 of the amino acids excluding glycine are L-type amino acids and the other amino acids are D-type amino acids.

2. The peptide according to claim 1, wherein the peptide comprises at least one of the following, in the case of comprising the amino acid sequence designated as SEQ ID NO: 1:
i) at least one of four histidines is a D-type amino acid;
ii) at least one of valines that are the third and eleventh amino acids, is an L-type amino acid or both are D-type amino acids;
iii) the fourth amino acid, serine, is a D-type amino acid;
iv) the eighth amino acid, glutamine, is a D-type amino acid; and
v) at least two of i through iv above are D-type amino acids.

3. The peptide according to claim 1, wherein the peptide comprises at least one of the followings, in the case of comprising the amino acid sequence designated as SEQ ID NO: 2:
i) at least one of four histidines is a D-type amino acid:
ii) at least one of valines that are at least the third and tenth amino acids, is an L-type amino acid or both are D-type amino acids;
iii) the fourth amino acid, serine, is a D-type amino acid; and
iv) at least two of i through iii are D-type amino acids.

4. The peptide according to claim 1, wherein
1) at least three of four histidines in a L-alloferon peptide designated as SEQ ID NO: 1 have been substituted with D-type histidines, and at least two among the two valines, serine the third amino acid, and glutamine the eighth amino acid are D-type amino acids; or
2) all the four histidines in a L-alloferon peptide designated as SEQ ID NO: 1 are D-amino acids and the eighth amino acid, glutamine, is either an L- or D-amino acid or have been deleted.

5. The peptide according to claim 1, comprising an amino acid sequence of any one of the amino acid sequences designated as SEQ ID NO: 3 through 14.

6. The peptide according to claim 1, comprising an amino acid sequence of any one of SEQ ID NO: 3 to 5, 7, 8, 10, and 12 to 14.

7. A pharmaceutical composition for the treatment of a neurodegenerative disease comprising a peptide of any one claim among claims 1 to 6 as an active ingredient.

8. The pharmaceutical composition according to claim 7, wherein the neurodegenerative disease is a neurodegenerative disease having the formation of abnormal protein aggregates as its etiology or pathology.

9. The pharmaceutical composition according to claim 8, wherein the abnormal protein aggregates are formed by the abnormal aggregation of a-synuclein, β-amyloid, TDP-34, p62, FUS protein, superoxide dismutase-1 (SOD-1), huntingtin protein, or tau protein.

10. The pharmaceutical composition according to claim 8, wherein the neurodegenerative disease is Alzheimer's disease (AD), Parkinson's disease (PD), Amyotrophic lateral sclerosis (ALS), Huntington's disease (HD), chronic traumatic encephalopathy (CTE), Lytico-bodig disease (LBD), frontotemporal lobar degeneration (FTD), corticobasal degeneration, or progressive supranuclear palsy.

11. A method of treating a subject suffering from a neurodegenerative disease, comprising administering a therapeutically effective amount of the peptide of any one claim among claims 1 to 6 to the subject.

12. A method of inhibiting the accumulation of pathogenic protein aggregates in the nervous system of a subject suffering from a neurodegenerative disease, comprising administering a therapeutically effective amount of the peptide of any one claim among claims 1 to 6 to the subject.
